# EUROPEAN PATENT SPECIFICATION

(11) **EP 4 221 651 B1**
(45) Date of publication and mention of the grant of the patent: **10.12.2025**
(21) Application number: 21794000.6
(22) Date of filing: 28.09.2021
(51) Int. Cl.: A61F 11/00

(54) **EAR CLEANING DEVICE**
OHRREINIGUNGSVORRICHTUNG
DISPOSITIF DE NETTOYAGE DES OREILLES

(30) Priority: 02.10.2020 IT 202000023245
(43) Date of publication of application: 09.08.2023
(73) Proprietor: Miocolibri Srls, 30175 Venezia (IT)
(72) Inventor: SERAFINI, Massimo Alessio, 04100 Latina (Roma) (IT); PAULIS, Alessandro, 20090 Segrate (Milano) (IT)
(74) Representative: Praxi Intellectual Property Milano
(86) International application number: PCT/IB2021/058830
(87) International publication number: WO 2022/070026

(56) References cited:
- EP-A1- 0 234 061
- EP-A1- 1 407 735
- DE-A1- 2 048 798
- DE-U1- 9 409 240

## Description

### TECHNICAL FIELD

The present invention relates to ear cleaning devices.

### STATE OF THE ART

As is known, the cleaning of the ears is essential to prevent infections and the formation of earwax plugs, and requires particular attention in the execution because it is not without risk to the parts of the ear, such as the eardrum, more internal than the auricle.

This cleaning is often carried out using a cotton swab (or Q-tip) which, however, has well-known contraindications, because it exposes the earwax to the risk of being pushed inside the ear canal and to the possibility of damaging the eardrum. Moreover, due to an incorrect disposal by the user, cotton buds accumulate in sewage treatment plants and water courses.

Several types of ear cleaning devices have been proposed to replace Q-tips.

One type of device involves the use of a soft, helical-shaped tip that is inserted into the ear and rotated (manually or electrically) to remove earwax.

Other types of devices, such as the so-called auricular syringe, require the introduction of water or emollient liquids into the ear canal.

A device has also been proposed that relies on the vibration (caused by an electric motor) of a tip introduced into the ear canal, which would cause fragmentation of the earwax, and subsequent aspiration of the same.

According to another typology, the device consists of a rod made of biocompatible plastic having at both ends a silicone head equipped with protuberances intended to facilitate the removal of earwax; this device is used in a manner similar to how the cotton bud is used.

Document CN105982788 describes a cleaning device comprising a pressure air pump connected to an internally hollow terminal body having soft hairs on its surface. In use, the terminal body is inserted into the ear canal and then pressure is applied to the pump causing said body to expand so that the soft hairs contact the walls of the ear canal removing ear wax. According to that document, after the terminal body is deflated, it is pulled out of the ear removing the earwax that remains trapped between the soft hairs.

Document DE2048798 describes an ear cleaning device comprising a rubber ball connected via a channel to a free end intended to expand when air is pushed from the rubber ball. Solid " warts " (indicated by 6 in document DE2048798) are disposed on the free end to capture earwax.

Document EP0234061 describes an instrument for cleaning the external ear canal consisting of a stem and a pear-shaped head. There is a set of protruding ribs on the head for removing earwax.

### SUMMARY OF THE INVENTION

The purpose of the present invention is to propose an ear cleaning device, based on the use of an air-expandable cleaning body, which is an alternative to the one known and mentioned above and which, in particular, is efficient in the cleaning action while also offering full safety for the user in its use.

It is an object of the present invention an ear cleaning device as defined by claim 1 and particular embodiments thereof described by dependent claims 2-10.

### BRIEF DESCRIPTION OF THE DRAWINGS

The constructional and functional features of the invention may be better understood from the following detailed description, in which reference is made to the accompanying drawing plates representing some preferred and non-limiting forms of implementation, wherein:
- figure 1 shows, by a side view and a de-assembled configuration, an ear cleaning device made according to a particular embodiment of the present invention;
- Figure 2 shows a front perspective view of said device, in the de-assembled configuration;
- Figure 3 shows a rear perspective view of said device, in the de-assembled configuration;
- Figure 4 shows a front perspective view of said device, in an assembled configuration and in rest conditions;
- Figure 5 shows a rear perspective view of said device, in the assembled configuration and in rest conditions;
- figure 6 shows a side view of said device, in assembled configuration and in rest conditions;
- Figure 7 shows, in a perspective view, a cleaning body of said device having a sectioned insertion portion;
- Figure 8 shows a side view of said device, in the assembled configuration and in a condition of expansion of said cleaning body;
- Figure 9 shows, in a side view, said cleaning body in an expanded condition.

### DETAILED DESCRIPTION

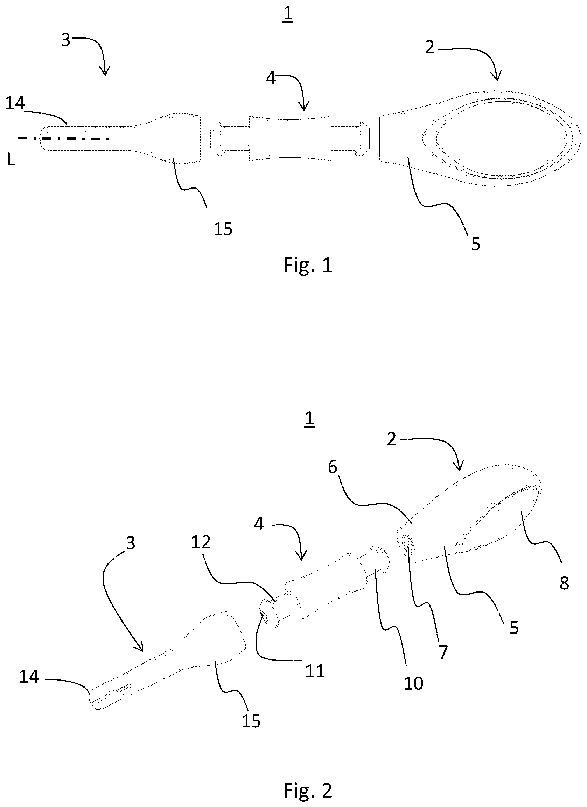

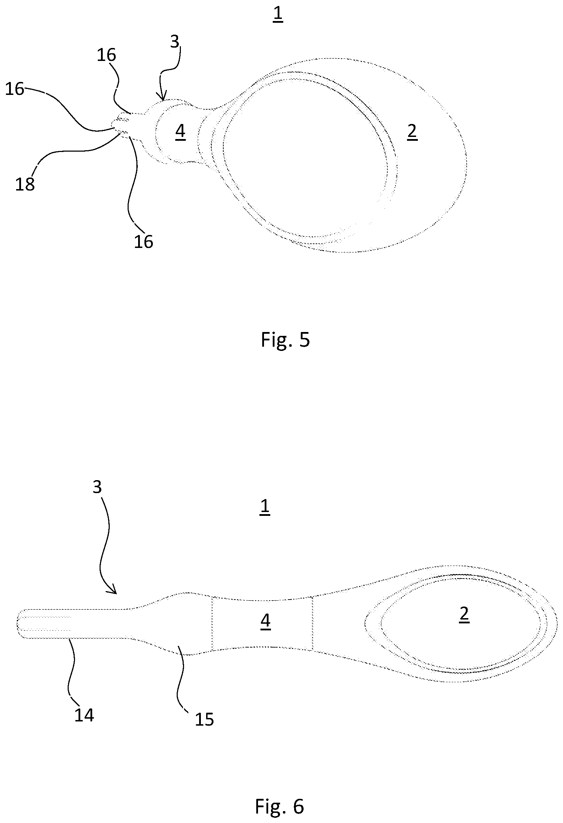

Figures 1-9 show an example of an ear cleaning device 1 comprising an air nozzle 2 and a cleaning body 3 connected to the air nozzle 2. According to the described example, the cleaning device 1 may further comprise a junction member 4 of the nozzle 2 to the cleaning body 3.

The dispenser 2 is configured to transfer pressurized air to the cleaning body 3. In accordance with a preferred embodiment, the air dispenser 2 is a manual pressure pump 2 comprising a small elastic bag (or ampoule) 5 having an end 6 provided with an air inlet/outlet hole 7 (Fig. 2).

The manual pressure pump 2 is, for example, made of an elastomeric material (such as, for example, latex or rubber) or biocompatible silicone. Advantageously, the outer wall of the elastic bag 5 is shaped to define pressure areas 8 intended to be squeezed between the user's fingers. The manual pressure pump 2 when squeezed delivers air through the hole 7 and, when the manual pressure has ceased, is refilled with air by an elastic expansion of the elastic bag 5.

Note that an air dispenser other than the described manual pressure pump may be provided. For example, a dispenser of the piston type, i.e., a syringe type, may be employed.

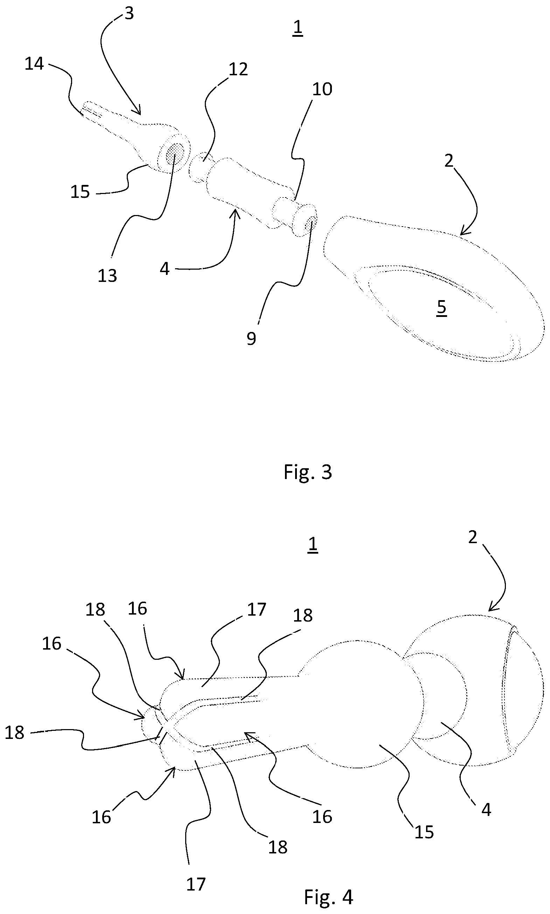

The junction element 4, if provided, internally defines an air connection duct open on a first hole 9 (Fig.3), at a first end 10, and a second hole 11 (Fig.2), at a second end 12. For example, the first end 10 is configured to be inserted into the hole 7 of the manual pressure pump 2 and the second end 12 is configured to be inserted into a further hole 13 provided by the cleaning body 3. According to a particular embodiment, the first and second ends 10 and 12 have a relative mushroom-shaped head so as to avoid an undesirable separation from the manual pressure pump 2 and the cleaning body 3.

It should be noted that the junction element 4 may be removably attached to the cleaning body 3 and the dispenser 2 in other ways than those described, and for example, such as providing for the use of ferrules, sealing bands or by screwing. Furthermore, it is also possible that the cleaning body 3 and the dispenser 2 may be directly coupled without the use of any joining element.

In particular, the junction element 4 is made of a rigid (preferably, hypoallergenic) plastic material such as, for example, polypropylene. Alternatively, so-called biodegradable plastics may be used.

The cleaning body 3 is internally hollow and is configured to be placed in communication with the air dispenser 2 so as to be able to receive inside itself the air supplied by the latter. This cleaning body develops in length according to a relative longitudinal axis L (Fig. 1).

The cleaning body 3 is such that it assumes a resting configuration in which it is at least partially insertable into the ear canal of one ear and an expanded configuration in which it presents an increased volume due to the air supplied by the nozzle 2. As will also be described later, it is in the expanded configuration that the cleaning body 3 will be able to exert its cleaning function of the ear canal.

In greater detail, the cleaning body 3 comprises an insertion portion 14 having an elongated shape, i.e., developing more along the longitudinal axis L (Fig. 1 and Fig. 2). In addition, the cleaning body 3 includes a fitting portion 15 (in one piece with the insertion portion 14) provided with the aforementioned further hole 13 for inserting the second end 12 of the junction element 4.

Note that the insertion portion 14 includes a plurality of lobes (or bulges) 16 that extend in the direction of the longitudinal axis L; that is, such lobes 16 extend lengthwise in the direction of the longitudinal axis L. Each lobe 16 defines a respective convex wall 17 (i.e., a bulge) that connects to intermediate walls 18 that extend in the direction of the longitudinal axis L and form corresponding grooves between the lobes 16 themselves. The grooves 18 extend in the direction of the longitudinal axis L but are not necessarily parallel to the axis L.

With respect to the number of lobes 16, the example in the figures shows the presence of four lobes 16, but cleaning portions 14 having two lobes, three lobes or a number of lobes greater than four, for example, six, seven or eight lobes, may also be employed.

The cleaning body 3 is soft in that it should be able to inflate, adapt to the shape of the ear canal as much as possible, and should not cause damage to the eardrum. In particular, the cleaning body 3 is made of an elastic material such as, for example, biocompatible silicone, or an elastomeric material such as, for example, latex or rubber. The cleaning body 3, or at least the insertion portion 14, may have a smooth outer wall or advantageously be provided with a suitable graininess or embossing that increases its abrasive power.

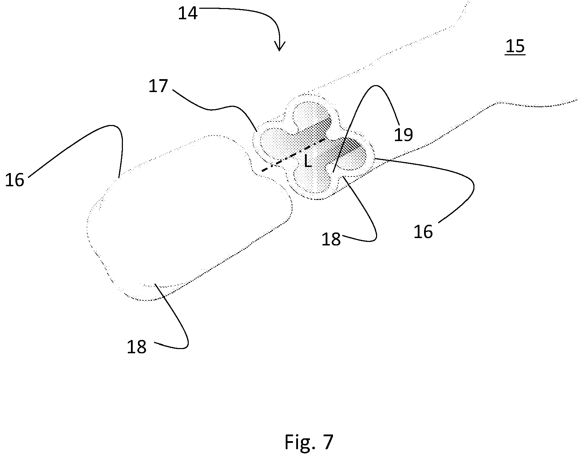

As shown, illustratively, in the sectioned embodiment of Figure 7, the insertion portion 14 is provided with internal ribs 19 running parallel to each intermediate wall 18 separating the lobes 16.

The above-described shape of the cleaning body 3 allows the lobes 16 to inflate, while the ribs 19 associated with the intermediate walls 18 resist the traction and torsion to which the device 1 is subjected in the expansion and cleaning action. The fitting portion 15 provides a seal with the fitting member 4 (if provided) or directly with the air nozzle 2, to prevent leakage of air from the interior of the cleaning device 1 to the exterior, with drops in internal pressure when inflated.

In addition, the lobe structure has been shown to allow an advantageous increase in the external expandable surface of the cleaning body 3 and also an increase in its abrasive function, compared to what can be obtained with a uniform, non-lobed structure.

In particular, the lobes 16, in extending towards the fitting portion 15 of the cleaning body 3, tend to merge with each other until they form a uniform overall shape, i.e. free of lobes and grooves. In the example considered, the fitting portion 15 of the cleaning body 3 has a spherical or cylindrical type outline (free of grooves and lobes), which is well suited to the function of connecting with the junction element 4.

Furthermore, the fitting portion 15 advantageously has a maximum diameter (distance from the longitudinal axis L) chosen so as to avoid a dangerous, excessive introduction of the cleaning body 3 into the ear canal. For example, the fitting portion 15 has a maximum diameter of about 10.0 mm (dimensions may vary depending on feasibility and the type of user: child, adult and small, medium or large animals).

With respect to possible size values, the insertion portion 14, in a resting configuration, may have, for example, a maximum diameter of between 4.5 mm and 5.00 mm, in particular, between 4.5 mm and 4.8 mm. Note that the values for the diameter of the resting portion of insertion 14 shown above are illustrative only and refer to use for the human ear. Keep in mind that the auditory cord has a fairly variable diameter of up to about 8-9 mm in the adult. Therefore, it is possible to envision different models of the cleaning device 1 having different dimensions.

For example, the thickness of the convex walls 17 (Figure 7) may be about 0.30 mm, while the overall thickness of the intermediate wall 18 with its associated rib 19 may be about 0.80 mm. In addition, the intermediate walls 18 may have a width of about 0.60 mm. Such values of the thicknesses are only illustrative as other values may be adopted also, but not only, depending on the number of lobes 16.

The ear cleaning device 1 can be easily assembled by the user by joining the air nozzle 2, the junction member 4, and the cleaning body so that it assumes the configuration shown in Figures 4-6. Advantageously, it is also possible to de-assemble the ear cleaning device 1 by separating its components, for example, to store it, to wash it, or to replace one of the components.

The ear cleaning device 1, assembled and in the resting configuration (i.e., with the cleaning body 3 un-expanded) is operated by the user grasping it by the manual pressure pump 2 and introducing the insertion portion 14 into the ear canal of one ear.

Next, the user presses his or her fingers on the hand pump 2 and pressurized air escapes from the hole 7 and passes through the inner conduit of the junction element 4 and enters the cleaning body 3.

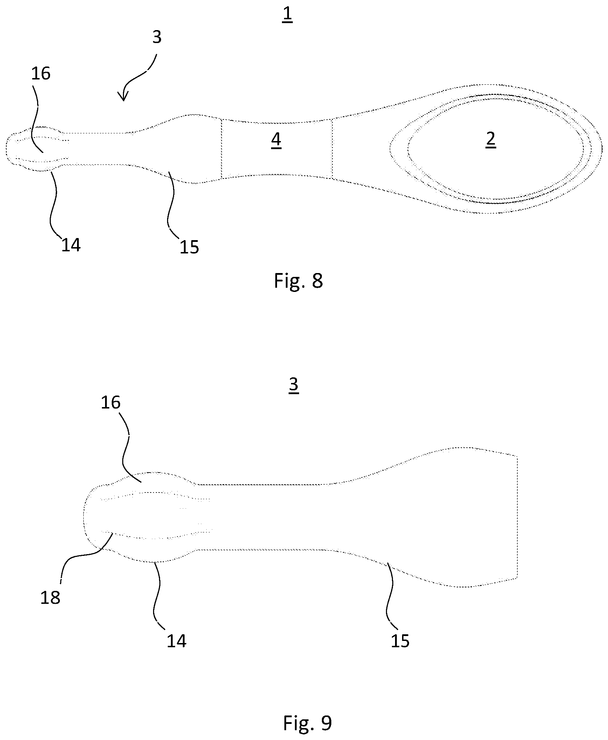

The air entering the cleaning body 3 causes an expansion (i.e., a bulge) of the insertion portion 14, which assumes the configuration shown, illustratively, in Figures 8 and 9. The insertion portion 14 when swollen adheres well to the ear canal of the ear. Fitting portion 15 does not expand significantly.

At this point, the user may perform rotations (e.g., in an alternative direction) or small translations of the cleaning device 1 so that the convex surfaces 17 of the swollen lobes 16 can effectively act by rubbing on the ear canal and remove the earwax.

The insertion portion 14, still in the expanded configuration, is extracted from the ear canal so that it removes the abrasion cerumen that has become fixed on its walls as a result of expansion and handling. The cleaning operation may be repeated several times.

Note that the cleaning operation described above may be preceded by the use of an ear canal cleanser.

Note that the lobes 16, together with the grooves 18, allow air to pass outwardly while avoiding both compressing air within the ear during introduction of the insertion portion 14 into the ear canal and creating decompression following removal of the insertion portion 14. This is important for protection of the eardrum.

The cleaning device 1 described is designed specifically for human ear cleaning, but the teachings provided can also be used to make an animal (e.g., dog or cat) ear cleaning device by tailoring the overall geometry of the device to the ear canal of the animal of interest.

It should be noted that although the above description refers to an air dispenser 2, it is also possible to alternatively employ a dispenser of a fluid other than air, such as may be water, but still such that the desired expansion of the insertion portion 14 can be achieved. For example, the aforementioned manual pressure pump or piston dispenser may also be used, with appropriate adaptations, to dispense water in a manner similar to that described for air.

The cleaning device 1 described above is very advantageous.

An advantage is attributable to the fact that the lobes 16 and the grooves 18 allow for adequate structural and sealing resistance during the air compression, device rotation and extraction phases. In addition, the presence of the inflated lobes, which constitute discontinuities, contributes favorably to the cleaning effect of the ear canal.

Another advantage concerns the safety for the user due to the aforementioned ability to avoid compressions or decompressions of air inside the ear canal that could, otherwise, be harmful.

The possibility of de-assembling the cleaning device 1 in order to wash it or replace part of its components is another advantageous aspect also because it favours a correct disposal of the component to be replaced.

### Legend of figure references

- ear cleaning device 1
- air dispenser 2
- junction element 4
- elastic bag 5
- tip 6
- hole 7
- pressure areas 8
- first hole 9
- first end 10
- second hole 11
- second end 12
- further hole 13
- insertion portion 14
- connecting portion 15
- lobes 16
- longitudinal axis L
- convex wall 17
- intermediate walls (grooves) 18
- ribs 19

## Claims

1. Ear cleaning device (1), including:
a fluid dispenser (2);
a cleaning hollow body (3) which is elongated according to a relative longitudinal axis (L) and in communication with the fluid dispenser (2), the cleaning body (3) including an insertion portion (14) configured to assume a resting configuration in which it is at least partially insertable in the ear canal and an expanded configuration in which it has an increased volume due to expansion caused by the fluid supplied by the dispenser (2);
**characterised by** the fact that said insertion portion (14) includes:
- a plurality of lobes (16) which are elongated according to the longitudinal axis and form convex portions (17) towards the outside of the insertion portion, wherein the lobes (16) are such that they inflate when the insertion portion (14) assumes the expanded configuration, and
- connecting walls (18) between the lobes (16) which develop along the longitudinal axis forming grooves between the lobes.

2. Device (1) according to claim 1, wherein the plurality of lobes includes a number of lobes equal to or greater than two.

3. Device (1) according to at least one of the preceding claims, wherein the insertion portion (14) assumes an ovoid shape in the expanded configuration; said grooves being configured to allow a flow of fluid from the ear canal to the outside of the ear.

4. Device (1) according to at least one of the preceding claims, wherein the cleaning body (3) includes a connection portion (15), in one piece with the insertion portion, and connectable to the fluid dispenser (2).

5. Device (1) according to at least one of the preceding claims, wherein the cleaning body is made of a material selected from the group: elastic material; biocompatible silicone, elastomer material, latex, rubber.

6. Device (1) according to claim 1 or 4, wherein:
the connection portion (15) has a diameter that prevents the insertion of the connection portion (15) into the ear canal.

7. Device (1) according to at least one of the preceding claims, wherein the fluid dispenser (2) includes a manual pressure air delivery pump with an elastic bag (5) on which the user can apply pressure.

8. Device (1) according to at least one of the preceding claims, further including a junction element (4) having an internal fluid passage pipe and a first end (10) connectable to said dispenser (2) and a second end (12) connectable to said cleaning body (3).

9. Device (1) according to at least one of the preceding claims, in which at least said fluid dispenser (2) and said cleaning body (3) are separable from each other and can be re-assembled.

10. Device (1) according to at least one of the preceding claims, in which said device is made with a shape suitable for cleaning human ears or ears of animals such as, for example, dogs and cats.

## Patentansprüche

1. Ohrreinigungsvorrichtung (1), einschließend:
einen Flüssigkeitsspender (2);
einen Reinigungshohlkörper (3), der gemäß einer relativen Längsachse (L) verlängert ist und mit dem Flüssigkeitsspender (2) in Verbindung steht, wobei der Reinigungskörper (3) einen Einführabschnitt (14), der konfiguriert ist, um eine Ruhekonfiguration einzunehmen, in der er mindestens teilweise in den Gehörgang eingeführt werden kann, und eine ausgedehnte Konfiguration einschließt, in der er aufgrund der Ausdehnung, die durch die von dem Spender (2) zugeführte Flüssigkeit verursacht wird, ein vergrößertes Volumen aufweist;
**gekennzeichnet durch** die Tatsache, dass der Einführabschnitt (14) einschließt:
- eine Vielzahl von Lappen (16), die gemäß der Längsachse verlängert sind und konvexe Abschnitte (17) zur Außenseite des Einführabschnitts hin bilden, wobei die Lappen (16) so beschaffen sind, dass sie sich aufblasen, wenn der Einführabschnitt (14) die ausgedehnte Konfiguration annimmt, und
- Verbindungswände (18) zwischen den Lappen (16), die sich entlang der Längsachse entwickeln und Rillen zwischen den Lappen bilden.

2. Vorrichtung (1) nach Anspruch 1, wobei die Vielzahl von Lappen eine Anzahl von Lappen einschließt, die gleich oder größer als zwei ist.

3. Vorrichtung (1) nach mindestens einem der vorstehenden Ansprüche, wobei der Einführabschnitt (14) in der ausgedehnten Konfiguration eine eiförmige Form annimmt; wobei die Rillen konfiguriert sind, um einen Flüssigkeitsfluss vom Gehörgang zur Außenseite des Ohrs zu ermöglichen.

4. Vorrichtung (1) nach mindestens einem der vorstehenden Ansprüche, wobei der Reinigungskörper (3) einen Verbindungsabschnitt (15) einschließt, der einstückig mit dem Einführabschnitt ist und mit dem Flüssigkeitsspender (2) verbunden werden kann.

5. Vorrichtung (1) nach mindestens einem der vorstehenden Ansprüche, wobei der Reinigungskörper aus einem Material hergestellt ist, das aus folgender Gruppe ausgewählt ist: elastisches Material; biokompatibles Silikon, Elastomermaterial, Latex, Gummi.

6. Vorrichtung (1) nach Anspruch 1 oder 4, wobei:
der Verbindungsabschnitt (15) einen Durchmesser aufweist, der das Einführen des Verbindungsabschnitts (15) in den Gehörgang verhindert.

7. Vorrichtung (1) nach mindestens einem der vorstehenden Ansprüche, wobei der Flüssigkeitsspender (2) eine manuelle Druckluft-Förderpumpe mit einem elastischen Beutel (5) einschließt, auf den der Benutzer Druck ausüben kann.

8. Vorrichtung (1) nach mindestens einem der vorstehenden Ansprüche, die ferner ein Verbindungselement (4) einschließt, das ein inneres Flüssigkeitsdurchgangsrohr und ein erstes Ende (10), das mit dem Spender (2) verbunden werden kann, und ein zweites Ende (12) aufweist, das mit dem Reinigungskörper (3) verbunden werden kann.

9. Vorrichtung (1) nach mindestens einem der vorstehenden Ansprüche, wobei mindestens der Flüssigkeitsspender (2) und der Reinigungskörper (3) voneinander getrennt und wieder zusammengesetzt werden können.

10. Vorrichtung (1) nach mindestens einem der vorstehenden Ansprüche, wobei die Vorrichtung eine Form aufweist, die für das Reinigen von menschlichen Ohren oder Ohren von Tieren, wie z. B. Hunden und Katzen, geeignet ist.

## Revendications

1. Dispositif de nettoyage des oreilles (1), comportant :
un distributeur de fluide (2) ;
un corps creux de nettoyage (3) qui est allongé selon un axe longitudinal relatif (L) et en communication avec le distributeur de fluide (2), le corps de nettoyage (3) comportant une partie d'insertion (14) conçue pour prendre une configuration de repos dans laquelle il est au moins partiellement insérable dans le conduit auditif et une configuration expansée dans laquelle il a un volume accru en raison de l'expansion causée par le fluide fourni par le distributeur (2) ;
**caractérisée par le fait que** ladite partie d'insertion (14) comporte :
- une pluralité de lobes (16) qui sont allongés selon l'axe longitudinal et forment des parties convexes (17) vers l'extérieur de la partie d'insertion, dans lequel les lobes (16) sont de telle sorte qu'ils se gonflent lorsque la partie d'insertion (14) prend la configuration expansée, et
- des parois de liaison (18) entre les lobes (16) qui se développent le long de l'axe longitudinal en formant des rainures entre les lobes.

2. Dispositif (1) selon la revendication 1, dans lequel la pluralité de lobes comporte un nombre de lobes égal ou supérieur à deux.

3. Dispositif (1) selon au moins l'une des revendications précédentes, dans lequel la partie d'insertion (14) prend une forme ovoïde dans la configuration expansée ; lesdites rainures sont conçues pour permettre un écoulement de fluide du conduit auditif vers l'extérieur de l'oreille.

4. Dispositif (1) selon au moins l'une des revendications précédentes, dans lequel le corps de nettoyage (3) comporte une partie de liaison (15), d'un seul tenant avec la partie d'insertion, et pouvant être reliée au distributeur de fluide (2).

5. Dispositif (1) selon au moins l'une des revendications précédentes, dans lequel le corps de nettoyage est constitué d'un matériau choisi parmi le groupe : matériau élastique ; silicone biocompatible, matériau élastomère, latex, caoutchouc.

6. Dispositif (1) selon la revendication 1 ou 4, dans lequel :
la partie de liaison (15) a un diamètre qui empêche l'insertion de la partie de liaison (15) dans le conduit auditif.

7. Dispositif (1) selon au moins l'une des revendications précédentes, dans lequel le distributeur de fluide (2) comporte une pompe à air à pression manuelle avec un sac élastique (5) sur lequel l'utilisateur peut exercer une pression.

8. Dispositif (1) selon au moins l'une des revendications précédentes, comportant en outre un élément de jonction (4) ayant un tuyau de passage de fluide interne et une première extrémité (10) pouvant être raccordée audit distributeur (2) et une seconde extrémité (12) pouvant être raccordée audit corps de nettoyage (3).

9. Dispositif (1) selon au moins l'une des revendications précédentes, dans lequel au moins ledit distributeur de fluide (2) et ledit corps de nettoyage (3) sont séparables l'un de l'autre et peuvent être réassemblés.

10. Dispositif (1) selon au moins l'une des revendications précédentes, dans lequel ledit dispositif est fabriqué avec une forme adaptée au nettoyage des oreilles humaines ou des oreilles d'animaux tels que, par exemple, les chiens et les chats.
